# EUROPEAN PATENT SPECIFICATION

(11) **EP 1 233 058 B1**
(45) Date of publication and mention of the grant of the patent: **20.12.2006**
(21) Application number: 00976285.7
(22) Date of filing: 16.11.2000
(51) Int. Cl.: C12N 5/02, C12N 5/08, A61K 35/26, A61P 35/00, C12Q 1/02

(54) **METHOD OF PROLIFERATING NATURAL KILLER CELLS**
VERFAHREN ZUR VERMEHRUNG VON NATüRLICHEN KILLERZELLEN
METHODE DE PROLIFERATION DE CELLULES TUEUSES NATURELLES

(30) Priority: 26.11.1999 JP 33607999
(43) Date of publication of application: 21.08.2002
(73) Proprietor: RIKEN, Wako-shi, Saitama 351-0198 (JP)
(72) Inventor: OHNO, Tadao, Riken, Tsukuba Institute, Tsukuba-shi, Ibaraki 305-0074 (JP); SAIJOH, Kaoru, Riken, Tsukuba Institute, Tsukuba-shi, Ibaraki 305-0074 (JP)
(74) Representative: Sternagel, Fleischer, Godemeyer & Partner
(86) International application number: PCT/JP2000/008079
(87) International publication number: WO 2001/038494

(56) References cited:
- WO-A1-97/39108
- FR-A- 2 775 692
- US-A- 5 415 874
- ISHIWATA I ET AL: "CARCINOEMBRYONIC PROTEINS PRODUCED BY WILMS' TUMOR CELLS IN-VITRO AND IN-VIVO" EXPERIMENTAL PATHOLOGY (JENA), vol. 41, no. 1, 1991, pages 1-9, XP009000730 ISSN: 0232-1513
- RABINOWICH H ET AL: "INCREASED PROLIFERATION LYTIC ACTIVITY AND PURITY OF HUMAN NATURAL KILLER CELLS COCULTURED WITH MITOGEN-ACTIVATED FEEDER CELLS" CELLULAR IMMUNOLOGY, vol. 135, no. 2, 1991, pages 454-470, XP001120159 ISSN: 0008-8749
- PIERSON B A ET AL: "HUMAN NATURAL KILLER CELL EXPANSION IS REGULATED BY THROMBOSPONDIN-MEDIATED ACTIVATION OF TRANSFORMING GROWTH FACTOR-BETA1 AND INDEPENDENT ACCESSORY CELL-DERIVED CONTACT AND SOLUBLE FACTORS" BLOOD, W.B. SAUNDERS, PHILADELPHIA, VA, US, vol. 87, no. 1, 1996, pages 180-189, XP000604748 ISSN: 0006-4971
- MANDELBOIM OFER ET AL: "Protection from lysis by natural killer cells of group 1 and 2 specificity is mediated by residue 80 in human histocompatibility leukocyte antigen C alleles and also occurs with empty major histocompatibility complex molecules." JOURNAL OF EXPERIMENTAL MEDICINE, vol. 184, no. 3, 1996, pages 913-922, XP009000703 ISSN: 0022-1007
- PORGADOR A. ET AL.: 'Natural killer cell lines kill autologous beta2-microglobulin-deficient melanoma cells: Implications for cancer immunotherapy' PROC. NATL. ACAD. SCI. USA vol. 94, 1997, pages 13140 - 13145, XP002937872

## Description

### Technical Field

The present invention relates to a method for producing human immunocytes.

### Background Art

Natural killer cells (hereinafter occasionally abbreviated as "NK cells" in the specification) in an animal body are lymphoid cells which participate in immune reactions. The cells have variety of functions, especially have strong activities for killing tumor cells, and therefore, it is considered that NK cells are one of important members in immunological surveillance mechanism in a living body for removing tumor cells or abnormal cells under tumor progression. For these reasons, studies to effectively utilize the cells for tumor therapy have been made since early days.

For example, when peripheral blood mononuclear cells (hereinafter occasionally abbreviated as "PBMC" in the specification) are cultured in a medium added with a large amount of interleukin-2 (hereinafter "interleukin" is occasionally abbreviated as "IL" in the specification) as one of lymphokines, so called lymphokine activiated killer lymphocytes (hereinafter occasionally abbreviated as "LAK cells" in the specification) will proliferate after about one week in a human system, and it is well known that the culture contains a large numbers of NK cells. LAK cells have been widely used for adoptive immunotherapy for tumor since the Rosenberg's study (Rosenberg, S.A., et al., N.Engl.J.Med.313, pp.1485-1492, 1985). LAK cells are also known to be effective for infectious diseases and they are focused as a tool for infectious diseases which are hardly curable by antibiotics. However, when LAK cells are cultured for a prolonged period of time, e.g., more than two weeks for mass proliferation of LAK cells, cytotoxic activity of NK cells may often significantly lowered. Accordingly, it is not desirable to obtain NK cells by LAK cell culture.

NK cells have an NK activity inhibitory receptor (hereinafter occasionally abbreviated as "NKIR" in the specification) on the surface, and the cytotoxic activity of NK cells is inhibited when some molecule binds to the receptor (Gumperz, J.E.and Parham, P., Nature, 378, pp.245-248, 1995). Generally, the class I major histocompatibility complex (MHC) class I molecules (hereinafter occasionally abbreviated as "MHC-I" in the specification) are expressed on the surface of normal cells and they play an important role in self or non-self recognition. Since some molecules that bind to NKIR exist among the class of MHC-I molecules, cytotoxic activity of NK cells are generally measured by using human chronic leukemia cell line K562 as a target cell, because K562 cell expresses almost no MHC-I molecule. In addition, it is known that NK cells are further activated and proliferated when they kill K562 cells.

In recent years, Group of Strominger developed a method for obtaining NK cells in large quantities, which comprises the steps of exposing human B cell line 721.221, expressing almost no MHC-I on the surface, to radiation for elimination of mitotic ability, conducting co-culture of the cells with PBMCs for 5 to 6 days, purifying NK cells from the culture, and further expanding the NK cells. It was revealed that NK cells obtained from a patient with melanoma by the above method were able to kill MHC-I negative autologous melanoma (Porgador, A., et al., Proc.Natl.Acad.Sci., USA, 94, pp.13140-13145, 1997). Although this method enables convenient proliferation of NK cells, the 721.221 cells used as the target of NK cells are not necessarily more efficient to stimulate proliferation of NK cells than K562 cells, because the cells actually express a very slight amount of MHC-I as K562 cells (Hay, R., American Type Culture Collection, personal communication).

As a method for measurement of cytotoxic activity of human NK cells, a method has been used as a standard which comprises the steps of allowing K562 cells as target cells incorporate radioactive an inorganic Cr-51 salt beforehand, and then measuring an amount of Cr-51 that is extracellularly released after the death of K562 cells by using an apparatus for measuring radioactivity. This method is highly sensitive and specific, because Cr-51 is released solely from K562 cells, in the side of cells to be killed, even if large numbers of the cells are added that act as the side of cells killing the target cells (i.e., effector cells). However, the above method has a fatal problem that the use of an radioactive substance is hazardous.

As a method for measuring activity of cytotoxic T lymphocyte (hereinafter occasionally abbreviated as "CTL" in the specification), the inventor of the present invention proposed crystal violet staining method (hereinafter occasionally abbreviated as "CV method" in the specification) using an adhesive cell as a target cell (Liu, S.Q., et al., Nature Medicine 1, pp.267-271, 1995). This method is extremely safe since no radioactive substance is required. The target cells are co-cultured with effector cells, and then these two types of cells are separated each other and surviving target cells are stained with crystal violet and determined by colorimetry.

However, because K562 cells are floating cells, it is very difficult to separate the target cells after mixed with effector cells which are also floating cells. Therefore, a problem arises that the CV method is not applicable to the measurement of activity of human NK cells. The CV method is practically applicable if a target cell is available which is adhesive and highly sensitive to human NK cells as the K562. However, no report in this respect has been published so far.

### Disclosure of the Invention

An object of the present invention is to provide a more effective method for proliferating NK cells than conventionally known methods. More specifically, the object of the present invention is to provide a method that enables selective proliferation of NK cells from PBMC in more than doubled effectiveness as compared to the conventional methods, and provides NK cells that maintain high cytotoxic activity. Another object of the present invention is to provide human NK cells produced by a method having the above characteristics, and to provide a method for measuring cytotoxic activity of human NK cells with higher safety than that of the conventional methods.

The inventors of the present invention conducted various studies to achieve the foregoing objects. As a result, they screened cell lines from numbers of human cultured cell lines, falling within a class of cells as targets of NK cells, which did not increase and expressed MHC-I molecules even when interferon γ (hereinafter occasionally abbreviated as "IFNg" in the specification), an expression inducer of MHC-I molecules, was added to the cell culture, and they found that remarkably higher stimulatory effect on NK cell proliferation as compared to K562 cells was obtained when a particular cell line among the above cell lines was used. The present invention was achieved on the basis of these findings.

The present invention thus provides a method for proliferating human natural killer cells, which comprises the step of conducting co-culture of peripheral blood mononuclear cells and human Wilms tumor cell line HFWT. According to preferred embodiments of the aforementioned invention, provided are the aforementioned method wherein autologous natural killer cells of an individual are proliferated by using peripheral blood mononuclear cells isolated from said human individual, preferably as a tumor patient; the aforementioned method wherein the human Wilms tumor cell line HFWT which is treated beforehand for elimination of proliferating ability is used; and the aforementioned method wherein the culture is conducted in the presence of interleukin-2.

For example, tumor patient's autologous natural killer cells with maintained high cytotoxic activity can be mass produced by culturing peripheral blood mononuclear cells isolated from peripheral blood of said patient with human Wilms tumor cell line HFWT in accordance with the method of the present invention. The present invention also provides human natural killer cells produced by the above method, and an agent for treatment a tumor for a tumor patient, which comprises patient's autologous natural killer cells proliferated by the above method using human peripheral blood mononuclear cells (PBMC) isolated from said tumor patient.

From another aspect, the present invention provides a method for measuring cytotoxic activity of human natural killer cells, which comprises the step of conducting co-culture of human Wilms tumor cell line HFWT and human natural killer cells, and staining surviving target cells after the culture. According to preferred embodiment of the aforementioned invention, provided is the aforementioned method wherein crystal violet is used for the staining. According to the aforementioned methods, the cytotoxic activity of human NK cells can be accurately and conveniently measured without using an radioactive substance.

### Brief Explanation of the Drawings

Fig. 1 shows the result obtained by conducting selective culture of NK cells using PBMC of healthy human individual 1 (age 40, female) according to the method of the present invention.
Fig. 2 shows the result obtained by conducting selective culture of NK cells using PBMC of healthy human individual 2 (age 27, male) according to the method of the present invention.
Fig. 3 shows the result obtained by conducting cytotoxic test using the cultured lymphocytes obtained in Experiment 3 of Example 1.
Fig. 4 shows effects of the addition of interleukins on selective culture of NK cells. In the figure, ILs shows the result where four kinds of interleukins were used for culture and IL-2 shows the result where only IL-2 was used as the interleukin.
Fig. 5 shows cytotoxic activities of lymphocytes containing NK cells obtained from healthy human individual 2 or 3 as an effect of the interleukin addition on the selective culture of NK cells.
Fig. 6 shows the result of selective expansion culture of NK cells according to the method of the present invention by using PBMC of a tumor patient.
Fig. 7 shows the result of cytotoxic test where expansion culture was conducted by using PBMC of a tumor patient and K562 cells or HFWT cells as a target cell.

### Best Mode for Carrying out the Invention

The present invention is characterized by conducting co-culture of human PBMC with human Wilms tumor cell line HFWT as a target cell in proliferation of human NK cells. According to the method of present invention, it is possible to selectively proliferate NK cells among PBMC. Human Wilms tumor cell line HFWT has been deposited in RIKEN Cell Bank (3-1-1, Koyadai, Tsukuba, Ibaraki, Japan) of The Institute of Physical and Chemical Research (RIKEN, 2-1, Hirosawa, Wako, Saitama, Japan) as accession number RCB0665, and the cell line is available on request.

In the method of the present invention, a culture method is not particularly limited, and generally, cells can be cultured in the manner described below. To an ordinary culture medium for animal cells, serum or plasma and a growth factor supporting for proliferation of lymphocytes are added, and PBMC isolated from human peripheral blood is further added to the mixture. The mixture is added to HFWT cells and the mixture is cultured. The kinds of serum or plasma added to the medium are not particularly limited, and any commercially available serum or plasma derived from various kinds of animals may be used. Serum or plasma which is human-derived and has blood type of AB is preferable, and serum or plasma derived from an individual from whom PBMC is collected is more preferable. A concentration may be about 1 to 30% by volume. A medium may also be used which is capable of supporting the proliferation of lymphocytes in a serum-free state.

As the growth factor, interleukin-2 (IL-2) is most preferable. A combination of cytokines which induce proliferation of lymphocytes from PBMC, lectins which stimulate proliferation of lymphocytes, or PBMC which is collected from a human individual different from a subject from whom PBMC is collected and further treated for elimination of proliferating ability may be used instead of the growth factor. The method for elimination of proliferating ability is not particularly limited, and method known to skilled artisans such as radiation exposure and Mytomicin C treatment may be used. A concentration of IL-2 to be added is preferably 10 - 2,000 IU/ml.

A ratio of PBMC and HFWT cell is not particularly limited. Approximately 10:1 is preferred. Since the target HFWT cell is adhesive, it is generally preferable to use the cell in a state of adhesion after treatment for elimination of proliferating ability. Alternatively, it is possible to use the cells in a state of suspension after physically stripping the cells in the adhesion state. Target HFWT cells are rapidly killed by NK cells, when the NK cells are activated and in fast proliferation state. When the target cells are used in that condition, it is not always necessary to treat the cells for elimination of proliferating ability. Period of time for the culture of PBMC is not particularly limited, and may preferably be more than 5 days. NK cells proliferates with maintained high activities. It is possible to continue the culture until the NK cells spontaneously lose their proliferating ability.

The method for measuring cytotoxic activity of human natural killer cells provided by the present invention is characterized by conducting co-culture of human Wilms tumor cell line HFWT and human natural killer cells, and then staining surviving target cells after the culture. This method can be generally performed in accordance with the known method by using NK cells as effector cells and HFWT cells as a target cell (Liu, S.Q., et al., Nature Medicine, 1, pp.267-271, 1995). It is preferable to use crystal violet for staining. Since HFWT cells are adhesive cultured cells, the activity of human NK cells can be measured accurately and conveniently by the method of the present invention without using an radioactive substance.

### Examples

The present invention will be more specifically explained with reference to examples. However, the scope of the present invention is not limited to these examples. The cell strains in the following examples were used after screening 209 human tumor cell lines which have been deposited to RIKEN Cell Bank. By flow cytometry technique well known to those skilled in the art, chronic myelogenous leukemia cell line K562 (cell No. RCB0027), Wilms tumor cell line HFWT (cell No. RCB0665), and melanoma cell line HMV-II (cell No. RCB0777) among the cell lines screened were verified to express almost no MHC-I molecule (in the latter two cell lines, a slight amount of expression of MHC-I were recognized in the same level as that of K562 cell), and to be absolutely free from enhancement of expression of MHC-I with treatment of IFNg added in the culture for 24 hours.

It is known that many variants derived from K562 cell are present, and the expression of MHC-I was reported to be induced by IFNg treatment in the original cell line (Lozzio, C.B. and Lozzio, B.B., Blood 45, pp.321-324, 1975). However, K562 cell line used in these examples is a variant from the original cell line, and the cell remains unchanged in the state of extremely low level expression of MHC-I even by IFNg treatment. Higashino cell line was derived from a brain tumor tissue determined as gliosarcoma by pathological diagnosis and subcultured in RIKEN Cell Bank. As the cells constantly express MHC-I and MHC-II strongly under an ordinary culture condition, the cells were used as a control for comparison with the cells which express almost no MHC-I.

### Example 1: Selective culture of NK cells of healthy individuals

Experiments were conducted to know whether or not NK cells can be selectively proliferated from PBMC of a healthy individual where HFWT cells were used as target cells.

### <Method>

### 1. Culture of target cells

1 × 10⁵ of target cells were inoculated on each well of 24-well plates. As the target cells, Higashino, HFWT, K562, and HMV-II were used, and as the medium, Ham F12 medium supplemented with 10% fetal bovine serum was used. After cultivation of these cells overnight, the cells were exposed to X-ray radiation of 50 Gy, washed with PBS(-) three times, and then used as target cells.

### 2. Preparation of PBMC

PBMC were isolated from 30 ml of peripheral blood of a healthy individual by a method well known to those skilled in the art (Kawai, K., et al., Cancer Immunol. Immunother. 35, pp.225-229, 1992). Plasma collected in this process was diluted to half of its concentration with PBS(-) and the dilution was used in the following culture. The concentration applied was 10% in volume ratio in the state of 1/2 dilution (that is 5% as plasma).

### 3. Culture of PBMC

1 × 10⁶ of PBMC suspended in 2 ml, per each well, of RHAM α medium (Kawai, K., et al., Cancer Immunol. Immunother. 35, pp.225-229, 1992) supplemented with plasma were inoculated on the target cells. IL-1 (final concentration 167 IU/ml, and in the same manner, concentrations hereinafter mean final concentrations), IL-2 (67 IU/ml), IL-4 (67 IU/well), IL-6 (134 IU/well) were added to the medium. The mixture was cultured in CO₂ incubator for 37°C.

### 4. Flow cytometry

The cultured floating cells were stained with fluorescence-labeled monoclonal antibody specifically binding to each surface antigen according to a method well known to those skilled in the art, and then the cells were analyzed by using FACS (Becton Dickinson). Among the monoclonal antibodies used in the experiments, anti-CD3 antibody was used for distinction of T cells, and anti-CD56 antibody was used for distinction of NK cells.

### 5. Count of cell number

The number of cells was counted by using Tatai hemocytometer.

### 6. Measurement of cytotoxic activities

In accordance with the method previously reported (Liu, S.Q., et al., Nature Medicine 1, pp.267-271, 1995), the cytotoxic activities of cultured lymphocytes were measured by the CV method. In the Examples of the specification, the absorbance of target cells was taken as 100% after staining was carried out immediately before the addition of the lymphocytes to the living target cells. After incubation for 24 hours, cytotoxic activities exceeded 100% in the well containing only target cells as a control (i.e., the well where the ratio of effector cells and target cells (hereinafter abbreviated as E/T ratio) was 0) due to the target cell proliferation, which suggests that target cells themselves were not damaged in the experiments.

### <Results>

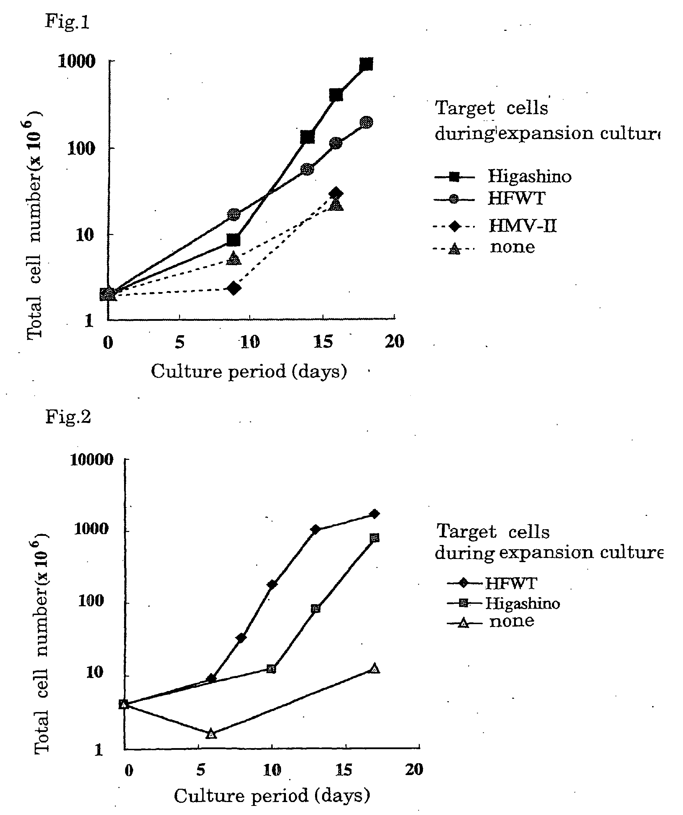

The results obtained by using PBMC of healthy individual 1 (age 40, female) are shown as experiments 1 and 2 in Table 1 as well as in Figure 1. The results obtained by using PBMC of healthy individual 2 (age 27, male) are shown as experiment 3 in Table 1 as well as in Figure 2.

**Table 1**

| Target cells | Ratio of cells having different surface antigen (%) | |
|---|---|---|
| | CD3+ CD56- | CD3- CD56+ |
| Experiment 1 (culture for 13 days) | | |
| none | 58.2 | 5.4 |
| Higashino | 96.0 | 0.2 |
| HFWT | 19.8 | 54.6 |
| K562 | 47.8 | 17.6 |

| Experiment 2 (culture for 14 days) | | |
|---|---|---|
| none | 60.9 | 13.0 |
| Higashino | 84.8 | 5.4 |
| HFWT | 7.3 | 75.9 |
| HMV-II | 55.0 | 18.9 |

| Experiment 3 (culture for 13 days) | | |
|---|---|---|
| none | 74.4 | 7.2 |
| Higashino | 95.1 | 1.7 |
| HFWT | 12.4 | 64.6 |

The combination of four kinds of ILs used in this Example is suitable for induction culture of CTL which has a property of simultaneously recognizing an MHC-I molecule and an antigenic peptide and killing the target cell (Liu, S.Q., et al., Cancer Immunol Immunotherapy, 39, pp.279-285, 1994). PBMC of a healthy individual usually contain 8 to 25% NK cells. As shown in Table 1, although the ratio of T cells shown as CD3+CD56- increased as high as 96% when Higashino cell was used as a target cell, NK cells shown as CD3-CD56+ became almost undetectable in experiment 1 and the ratio of the cells was 5.4% in experiment 2. In contrast, when HFWT cells in which almost no MHC-I molecule is present on the surface were used as target cells, the ratio of T cells was significantly decreased and the ratio of NK cells increased as high as 54.6%. This ratio is apparently higher than the result (17.6%) where K562 cell was used as a target cell in which similarly almost no MHC-I molecule is present on the surface. Experiment 2 also gave the same tendency, and the ratio of NK cells increased up to 75.9% when HFWT cell was used as a target cell, and the ratio is apparently higher than the result (18.9%) wherein HMV-II cell was used as a target cell in which similarly almost no MHC-I molecule is present on the surface.

Immediately after PBMC was prepared from peripheral blood of healthy individual 2, the ratio of T cells represented by CD3+CD56- was 55.4% and the ratio of NK cells represented by CD3-CD56+ was 13.2%. As shown in experiment 3 in Table 1, after 13 days of the culture, 95.1% of cells was occupied by T cells and NK cells almost disappeared when Higashino cells were used as target cells, whilst the ratio of NK cells increased as high as 64.6% when HFWT cells were used as target cells. Moreover, as shown in Figure 1, the cell proliferation derived from PBMC of healthy individual 1 was apparently higher when Higashino cells and HFWT cells were used as target cells as compared to the results obtained by using HMV-II cells as target cells or by using no target cell. In healthy individual 2 (Figure 2), the cells derived from PBMC proliferated more effectively when HWFT cells were used as target cells than when Higashino cells were used as target cells.

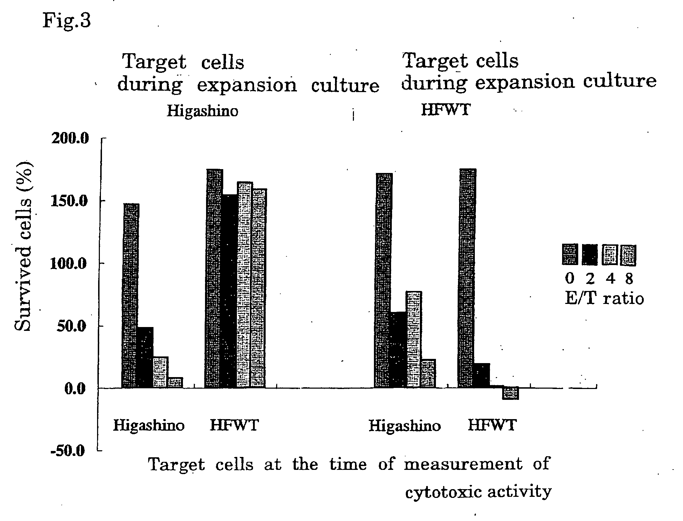

Further, the results of the cytotoxic test using cultured lymphocytes obtained in experiment 3 are shown in Figure 3. It was expected that the cultured lymphocytes obtained by using Higashino cells as target cells differentiated to CTL, since the cells were cultured in the presence of the combination of four kinds of ILs. In fact, these lymphocytes were highly effective in killing Higashino cells which were alive as the target cells at the time of measurement of cytotoxic activity. Under the condition that target cells proliferated up to 147% after 24 hours when E/T ratio was 0 (i.e., only target cells were present), the surviving target cells became 47.6% when E/T ratio was 2. The surviving target cells became only 7.5% when E/T ratio was 8. It was revealed that a higher ratio of surviving cells was obtained by lowering the E/T ratio, and a clear dose-dependent correlation was recognized. However, when the target cells at the time of measurement of cytotoxic activity was living HFWT cells, lymphocytes obtained by using Higashino cells as target cells completely failed to kill HFWT cell, which verified that the cultured lymphocytes obtained were typical CTL.

Whilst the cultured lymphocytes obtained by using HFWT cells as target cells were highly effective in killing living HFWT cells. The surviving target cells became 18.5% when E/T ratio was 2. The surviving target cells decreased as low as -9.2% when E/T ratio was 8 (the minus value was due to measurement error). It was revealed that a higher ratio of surviving cells was obtained by lowering the E/T ratio, which indicates the presence of a dose-dependent correlation. Accordingly, the cytotoxic activity of NK cells can be measured accurately by using HFWT cells as target cells which are alive at the time of measurement of cytotoxic activity.

It is also presumed that a slight amount of CTL other than NK cells was contained in the culture, because the cultured lymphocytes contained 12.4% T cells and they killed living Higashino cells, although the degree was apparently lower as compared to that of living HFWT cells. The surviving target cells became 60.5% when E/T ratio was 2, and the surviving target cells became 21.8% when E/T ratio was 8. Accordingly, as shown by these experiments, NK cells which maintain high cytotoxic activity can be selectively and efficiently proliferated from PPMC by using HFWT cells as target cells.

### Example 2 : Interleukins required for selective culture of NK cells

In Example 1, it was revealed that NK cells was selectively proliferated from PBMC of healthy individuals by using HFWT cells as target cells from which proliferating ability was eliminated. In the culture, four kinds of ILs were used. In this Example, the selective culture of NK cells was conducted by using HFWT cells as target cells in a medium supplemented with only IL-2 which is considered to be minimum essential for lymphocytes. The procedures were the same as those in Example 1 except that the kind of ILs to be added in the medium was changed. A concentration for sole addition of IL-2 was 500IU/ml. When cells cultured were examined by flow cytometry, anti-CD 16 antibody was used in addition to anti CD-56 antibody for distinction of NK cells.

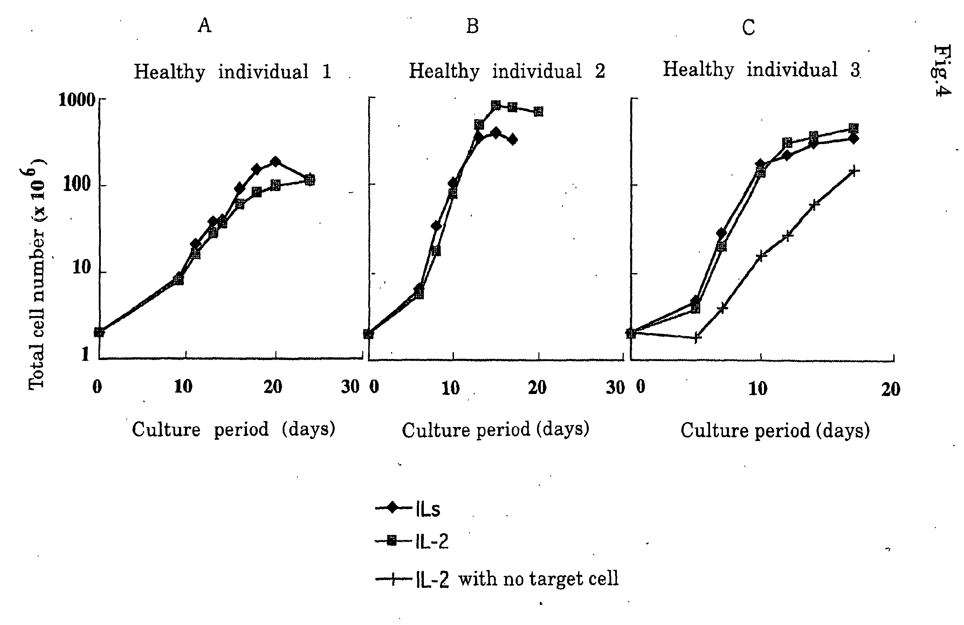

As shown in Figure 4, each PBMC derived from healthy individual 1, healthy individual 2, or healthy individual 3 (age 56: male) proliferated almost in the same level with addition of four kinds of ILs and with sole addition of IL-2 when HFWT cells were used as target cells. As shown in Figure 4(C), for PBMC derived from healthy individual 3, cell proliferation was much delayed when no target cell was used and only IL-2 was added to the medium.

In PBMCs of healthy individual 1, the ratio of NK cells represented by CD3-CD56+, as low as 8.0% at the start of the culture, reached to 77.3% after 17 days of the culture added solely with IL-2 as an IL when re-stimulation was carried out by addition of the target cells after 14 days of the culture. In PBMC of healthy individual 2 without the above re-stimulation, the ratio of NK cells represented by CD3-CD56+ was 90.7%, the ratio of NK cells represented by CD16+ was 79.1%, and the ratio of NK cells represented by CD16+CD56+ was 78.2% even in the culture added solely with IL-2. In also healthy individual 3, such tendency was observed, and the ratio of NK cells represented by CD3-CD56+ was 74.9%, the ratio of NK cells represented by CD16+ was 76.5%, and the ratio of NK cells represented by CD16+CD56+ was 75.7% even in the culture added solely with IL-2.

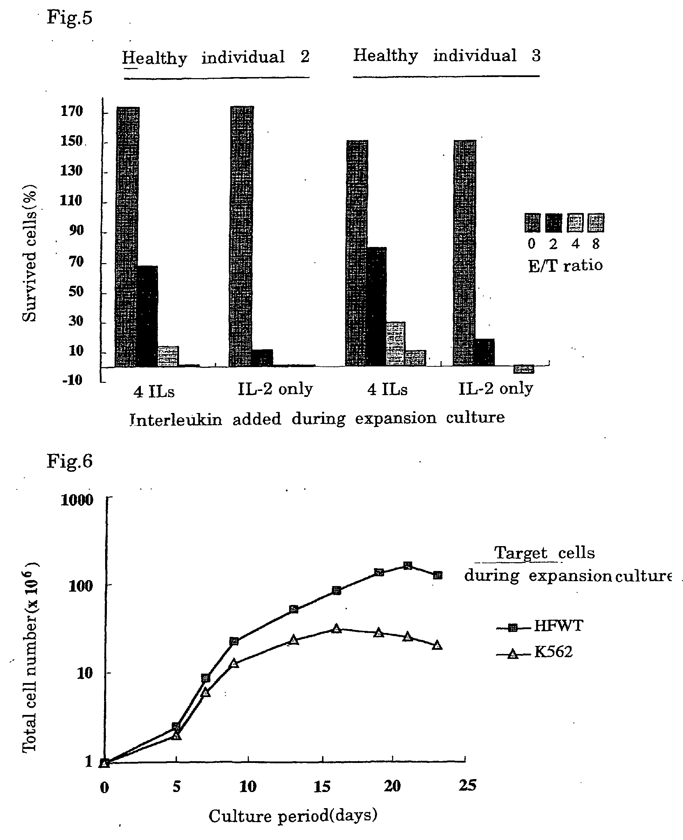

Fig. 5 shows results of cytotoxic activities of lymphocytes deriving from healthy individuals 2 and 3 in which most of the cells were NK cells. The living target cells used in the experiments were HFWT cells. Lymphocytes deriving both from healthy individual 2 and 3 gave sufficiently strong activity of NK cells, and therefore, only IL-2 may be sufficient as an IL added for selective culture of NK cells from PBMC by using HFWT cells as target cells, which enables selective and effective proliferation of NK cells which maintain high cytotoxic activity.

### Example 3 : Selective expansion culture of NK cells from PBMC of a tumor patient

In Examples 1 and 2, PBMC deriving from healthy individual were used. In this example, studies were made to know whether or not selective expansion culture of NK cells is possible by using PBMC of a tumor patient. The experiment was conducted according to the procedures in Example 1, except that HFWT cells or K562 cells were used as target cells in the culture of PBMC, and IL-2 was solely added to the culture as an IL at a concentration of 500 IU/ml. When cultured cells were examined by flow cytometry, anti CD16 antibody was used in addition to anti-CD56 antibody. The subject patient (age 51, male) was suffered from a brain tumor diagnosed as gliosarcoma and was the same individual from whom the Higashino cells used in Example 1 and 2 were collected.

The results are shown in Figure 6. As for proliferation of lymphocytes derived from PBMC, HFWT cells gave superior result as target cells to K562 cells. Starting from 1 × 10⁶ cells, the maximum number of accumulated and proliferated lymphocytes reached to 15.6 × 10⁷ when HFWT cells were used as the target, whereas 3.05 × 10⁷ when K562 cell was the target. The constitutions of cell populations derived from PBMC are shown in Table 2.

**Table 2**

| Target cells | Ratio of cells having different surface antigen (%) | | |
|---|---|---|---|
| | CD3+ CD56- | CD3-CD56+ | CD16+CD56+ |
| (cultured for 13 days) | | | |
| HFWT | 0.4 | 83.8 | 54.7 |
| K562 | 1.9 | 81.5 | 51.7 |

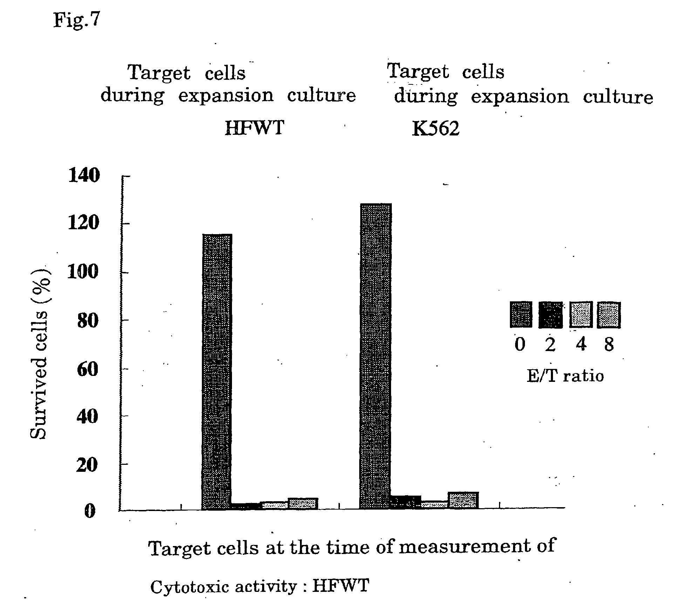

Both of expansion cultures where K562 cells were used as targets and where HFWT cells were used as targets gave almost same results, i.e., the ratio of NK cells represented by CD3-CD56+ was about 82 % and the ratio of NK cells represented by CD16+CD56+ was about 53 %. The cytotoxic activity of the resulting lymphocytes was measured by using HFWT cells as targets, and it was found that each of lymphocytes obtained by expansion culture by using K562 cells or HFWT cells as target cells gave potent cytotoxic activity (Figure 7).

### Industrial Applicability

According to the present invention, more effective expansion culture of human NK cells than conventional methods is achieved, and a large amount of NK cells can be easily obtained. NK cells obtained by the present invention by using PBMC of a tumor patient as a raw material can be applicable for treatments of tumors or infectious diseases, because the cells can be returned to a body of the subject patient without a risk of rejection and the like.

## Claims

1. A method for proliferating a human natural killer cell, which comprises the step of conducting a co-culture of a peripheral blood mononuclear cell and human Wilms tumor cell line HFWT.

2. The method according to claim 1, wherein an autologous natural killer cell of a human individual is proliferated by using a peripheral blood mononuclear cell isolated from said human individual.

3. The method according to claim 2, wherein the human individual is a tumor patient.

4. The method according to any one of claims 1 to 3, wherein the human Wilms tumor cell line HFWT which is subjected beforehand to elimination of proliferating ability is used.

5. The method according to any one of claims 1 to 4, wherein the culture is conducted in the presence of interleukin-2.

6. A human natural killer cell obtainable by the method according to any one of claims 1 to 5.

7. An agent for treatment of a tumor for a tumor patient which comprises an autologous natural killer cell of said patient which is proliferated by a method according to claim 1 using a peripheral blood mononuclear cell isolated from said tumor patient.

8. A method for measuring cytotoxic activity of a human natural killer cell, which comprises the step of conducting co-culture of human Wilms tumor cell line HFWT and a human natural killer cell, and then staining a surviving target cell after the culture.

9. The method according to claim 8, wherein crystal violet is used for staining.

## Patentansprüche

1. Ein Verfahren zur Vermehrung einer menschlichen natürlichen Killerzelle, umfassend den Schritt der Kokultivierung mononukleärer Zellen des peripheren Blutes und Zellen der menschlichen Wilms-Tumorzelllinie HFWT.

2. Das Verfahren gemäß Anspruch 1, **dadurch gekennzeichnet, dass** eine autologe natürliche Killerzelle eines menschlichen Individuums vermehrt wird unter Verwendung von mononukleären Zellen des peripheren Blutes, die aus dem besagten menschlichen Individuum isoliert wurden.

3. Das Verfahren gemäß Anspruch 2, **dadurch gekennzeichnet, dass** das menschliche Individuum ein Krebspatient ist.

4. Das Verfahren gemäß irgend einem der Ansprüche 1 bis 3, **dadurch gekennzeichnet, dass** die Fähigkeit zur Vermehrung der verwendeten menschlichen Wilms-Tumorzelllinie HFWT zuvor zerstört wurde.

5. Das Verfahren gemäß irgend einem der Ansprüche 1 bis 4, **dadurch gekennzeichnet, dass** die Kultivierung in Anwesenheit von Interleukin-2 durchgeführt wird.

6. Eine menschliche natürliche Killerzelle, erhältlich nach dem Verfahren gemäß irgend einem der Ansprüche 1 bis 5.

7. Ein Agens zur Behandlung eines Tumors für einen Tumorpatienten, umfassend eine autologe natürliche Killerzelle des besagten Patienten, die durch ein Verfahren gemäß Anspruch 1 vermehrt wurde unter Verwendung einer mononukleären Zelle des peripheren Blutes, die aus dem besagten Tumorpatienten isoliert wurde.

8. Ein Verfahren zur Messung der cytotoxischen Aktivität einer menschlichen natürlichen Killerzelle, umfassend den Schritt der Kokultivierung der menschlichen Wilms-Tumorzelllinie HFWT und einer menschlichen natürlichen Killerzelle, wobei anschließend die überlebende Zielzelle nach der Kultivierung angefärbt wird.

9. Das Verfahren gemäß Anspruch 8, **dadurch gekennzeichnet, dass** Kristallviolett zur Anfärbung verwendet wird.

## Revendications

1. Procédé de prolifération d'une cellule tueuse naturelle humaine, qui comprend l'étape consistant à réaliser une co-culture d'une cellule mononucléaire du sang périphérique et d'une lignée de cellules humaines de la tumeur de Wilms (HFWT).

2. Procédé selon la revendication 1, dans lequel une cellule tueuse naturelle autologue d'un individu humain est mise à proliférer en utilisant une cellule mononucléaire du sang périphérique isolée chez ledit individu humain.

3. Procédé selon la revendication 2, dans lequel l'individu humain est un patient atteint d'une tumeur.

4. Procédé selon l'une quelconque des revendications 1 à 3, dans lequel la lignée de cellules humaines de la tumeur de Wilms HFWT qui est auparavant soumise à une élimination de sa capacité de prolifération est utilisée.

5. Procédé selon l'une quelconque des revendications 1 à 4, dans lequel la culture est réalisée en présence d'interleukine 2.

6. Cellule tueuse naturelle humaine pouvant être obtenue par le procédé selon l'une quelconque des revendications 1 à 5.

7. Agent pour le traitement d'une tumeur pour un patient atteint d'une tumeur qui comprend une cellule tueuse naturelle autologue dudit patient qui est mise à proliférer par un procédé selon la revendication 1, en utilisant une cellule mononucléaire du sang périphérique isolée chez ledit patient atteint d'une tumeur.

8. Procédé pour mesurer l'activité cytotoxique d'une cellule tueuse naturelle humaine, qui comprend l'étape consistant à réaliser une co-culture d'une lignée de cellules humaines de la tumeur de Wilms HFWT et d'une cellule tueuse naturelle humaine, et ensuite à colorer une cellule cible survivante après la culture.

9. Procédé selon la revendication 8, dans lequel le cristal violet est utilisé pour colorer.
